# EUROPEAN PATENT SPECIFICATION

(11) **EP 1 285 075 B1**
(45) Date of publication and mention of the grant of the patent: **28.02.2007**
(21) Application number: 01931575.3
(22) Date of filing: 06.04.2001
(51) Int. Cl.: C12N 15/70, C12N 15/77, C12P 13/08, C12N 1/21

(54) **PROCESS FOR THE FERMENTATIVE PREPARATION OF L-THREONINE**
VERFAHREN ZUR FERMENTATIVEN HERSTELLUNG VON L-THREONINE
PROCEDE DE PREPARATION PAR FERMENTATION DE L-THREONINE

(30) Priority: 27.05.2000 DE 10026494; 23.01.2001 DE 10102823
(43) Date of publication of application: 26.02.2003
(73) Proprietor: Degussa GmbH, 40474 Düsseldorf (DE)
(72) Inventor: RIEPING, Mechthild, 33619 Bielefeld (DE)
(86) International application number: PCT/EP2001/003980
(87) International publication number: WO 2001/092545

(56) References cited:
- EP-A- 1 085 091
- WO-A-98/04715
- WO-A-99/53035
- MIZUKAMI T ET AL: "IMPROVEMENT OF THE STABILITY OF RECOMBINANT PLASMIDS CARRYING THE THREONINE OPERON IN AN L-THREONINE-HYPERPRODUCING STRAIN OF ESCHERICHIA COLI W" AGRICULTURAL AND BIOLOGICAL CHEMISTRY, JAPAN SOC. FOR BIOSCIENCE, BIOTECHNOLOGY AND AGROCHEM. TOKYO, JP, vol. 50, no. 4, 1986, pages 1019-1028, XP002047573 ISSN: 0002-1369
- EIKMANNS B J ET AL: "MOLECULAR ASPECTS OF LYSINE, THREONINE, AND ISOLEUCINE BIOSYNTHESIS IN CORYNEBACTERIUM GLUTAMICUM" ANTONIE VAN LEEUWENHOEK, DORDRECHT, NL, vol. 64, no. 2, 1993, pages 145-163, XP000918559
- KRAMER R: "Genetic and physiological approaches for the production of amino acids" JOURNAL OF BIOTECHNOLOGY, ELSEVIER SCIENCE PUBLISHERS, AMSTERDAM, NL, vol. 45, no. 1, 12 February 1996 (1996-02-12), pages 1-21, XP004036833 ISSN: 0168-1656

## Description

This invention relates to a process for the fermentative preparation of L-threonine using Enterobacteriaceae in which the thrE gene of coryneform bacteria is overexpressed.

### Prior art

L-Threonine is used in animal nutrition, in human medicine and in the pharmaceuticals industry. It is known that L-threonine can be prepared by fermentation of strains of Enterobacteriaceae, in particular Escherichia coli and Serratia marcescens. Because of their great importance, work is constantly being undertaken to improve the preparation processes. Improvements to the process can relate to fermentation measures, such as e.g. stirring and supply of oxygen, or the composition of the nutrient media, such as e.g. the sugar concentration during the fermentation, or the working up to the product form, by e.g. ion exchange chromatography, or the intrinsic output properties of the microorganism itself.

Methods of mutagenesis, selection and mutant selection are used to improve the output properties of these microorganisms. Strains which are resistant to antimetabolites, such as e.g. the threonine analogue α-amino-β-hydroxyvaleric acid (AHV), or are auxotrophic for amino acids of regulatory importance and produce L-threonine are obtained in this manner.

Methods of the recombinant DNA technique have also been employed for some years for improving the strain of Enterobacteriaceae strains which produce L-threonine, by amplifying individual threonine biosynthesis genes and investigating the effect on the L-threonine production.

WO 98/04715A discloses a process for producing L-threonine comprising culturing a strain of Escherichia coli that contains on the chromosome at least one threonine operon operably linked with at least one non-native promoter and recovering the L-threonine that is produced by said E.coli. The thrE gene is not overexpressed

### Object of the invention

The inventors had the object of providing new measures for improved fermentative preparation of L-threonine.

### Description of the invention

The invention provides a process for the fermentative preparation of L-threonine using Enterobacteriaceae which in particular already produce L-threonine and in which the thrE nucleotide sequence of coryneform bacteria which codes for the threonine export protein is, over-expressed.

In particular, the process is a process for the preparation of L-threonine, which comprises carrying out the following steps:
a) fermentation of microorganisms of the family Enterobacteriaceae in which at least the thrE gene of coryneform bacteria is over-expressed, optionally in combination with further genes,
b) concentration of the L-threonine in the medium or in the cells of the microorganisms of the family Enterobacteriaceae, and
c) isolation of the L-threonine.

The term "enhancement" in this connection describes the increase in the intracellular activity of one or more enzymes or proteins in a microorganism which are coded by the corresponding DNA, for example by increasing the number of copies of the gene or genes, using a potent promoter or a gene which codes for a corresponding enzyme or protein with a high activity, and optionally combining these measures.

The microorganisms which the present invention provides can prepare L-threonine from glucose, sucrose, lactose, fructose, maltose, molasses, starch, cellulose or from glycerol and ethanol. They are representatives of Enterobacteriaceae, in particular of the genera Escherichia and Serratia. Of the genus Escherichia the species Escherichia coli and of the genus Serratia the species Serratia marcescens are to be mentioned in particular.

Suitable L-threonine-producing strains of the genus Escherichia, in particular of the species Escherichia coli, are, for example
Escherichia coli TF427
Escherichia coli H4578
Escherichia coli KY10935
Escherichia coli VNIIgenetika MG-442
Escherichia coli VNIIgenetika M1
Escherichia coli VNIIgenetika 472T23
Escherichia coli BKIIM B-3996
Escherichia coli kat 13
Escherichia coli KCCM-10132

Suitable L-threonine-producing strains of the genus Serratia, in particular of the species Serratia marcescens, are, for example
Serratia marcescens HNr21
Serratia marcescens TLr156
Serratia marcescens T2000

It has been found that Enterobacteriaceae produce L-threonine in an improved manner after over-expression of the thrE gene of coryneform bacteria which codes for threonine export protein.

Nucleotide sequences of the thrE gene of coryneform bacteria are shown in SEQ ID No 1 and SEQ ID No 3 and the resulting amino acid sequences of the export proteins are shown in SEQ ID No 2 and SEQ ID No 4.

The thrE gene shown in SEQ ID No 1 and SEQ ID No 3 can be used according to the invention. Alleles of the thrE gene of coryneform bacteria which result from the degeneracy of the genetic code or due to sense mutations of neutral function can furthermore be used.

To achieve an over-expression, the number of copies of the corresponding genes can be increased, or the promoter and regulation region or the ribosome binding site upstream of the structural gene can be mutated. Expression cassettes which are incorporated upstream of the structural gene act in the same way. By inducible promoters, it is additionally possible to increase the expression in the course of fermentative L-threonine production. The expression is likewise improved by measures to prolong the life of the m-RNA. Furthermore, the enzyme activity is also increased by preventing the degradation of the enzyme protein. The genes or gene constructions can either be present in plasmids with a varying number of copies, or can be integrated and amplified in the chromosome. Alternatively, an over-expression of the genes in question can furthermore be achieved by changing the composition of the media and the culture procedure.

Instructions in this context can be found by the expert, inter alia, in Chang and Cohen (Journal of Bacteriology 134:1141-1156 (1978)), in Hartley and Gregori (Gene 13:347-353 (1981)), in Amann and Brosius (Gene 40:183-190 (1985)), in de Broer et al. (Proceedings of the National of Sciences of the United States of America 80:21-25 (1983)), in LaVallie et al. (BIO/TECHNOLOGY 11, 187-193 (1993)), in PCT/US97/13359, in Llosa et al. (Plasmid 26:222-224 (1991)), in Quandt and Klipp (Gene 80:161-169 (1989)), in Hamilton (Journal of Bacteriology 171:4617-4622 (1989), in Jensen and Hammer (Biotechnology and Bioengineering 58, 191-195 (1998) and in known textbooks of genetics and molecular biology.

Plasmid vectors which are capable of replication in Enterobacteriaceae, such as e.g. cloning vectors derived from pACYC184 (Bartolomé et al.; Gene 102, 75-78 (1991)), pTrc99A (Amann et al.; (Gene 69:301-315 (1988)), or pSC101 derivatives (Vocke and Bastia, Proceedings of the National Academy of Science USA 80 (21) : 6557-6561 (1983)) can be used. A strain transformed with a plasmid vector where the plasmid vector carries the thrE nucleotide sequence of coryneform bacteria which codes for the threonine export protein can be employed in a process according to the invention.

In addition, it may be advantageous for the production of L-threonine with strains of the family Enterobacteriaceae to over-express one or more enzymes of the known threonine biosynthesis pathway or enzymes of anaplerotic metabolism or enzymes for the production of reduced nicotinamide adenine dinucleotide phosphate, in addition to the thrE gene of coryneform bacteria. Thus, for example
- at the same time the thrABC operon which codes for aspartate kinase, homoserine dehydrogenase, homoserine kinase and threonine synthase (US-A-4,278,765), or
- at the same time the pyc gene which codes for pyruvate carboxylase (DE-A-19 831 609), or
- at the same time the pps gene which codes for phosphoenol pyruvate synthase (Molecular and General Genetics 231:332 (1992)), or
- at the same time the ppc gene which codes for phosphoenol pyruvate carboxylase (Gene 31:279-283 (1984)), or
- at the same time the genes pntA and pntB which code for transhydrogenase (European Journal of Biochemistry 158:647-653 (1986)), or
- at the same time the gdhA gene which codes for glutamate dehydrogenase (GGene 27:193-199 (1984)), or
- at the same time the rhtB gene which imparts L-homoserine resistance (EP-A-0994190)
can be over-expressed.

In addition to over-expression of the thrE gene it may furthermore be advantageous, for the production of L-threonine, to eliminate undesirable side reactions, such as e.g. threonine dehydrogenase (Nakayama: "Breeding of Amino Acid Producing Microorganisms", in: Overproduction of Microbial Products, Krumphanzl, Sikyta, Vanek (eds.), Academic Press, London, UK, 1982 and Bell and Turner, Biochemical Journal 156, 449-458 (1976)). Bacteria in which the metabolic pathways which reduce the formation of L-threonine are at least partly eliminated can be employed in the process according to the invention.

The microorganisms produced according to the invention can be cultured in the batch process (batch culture) or in the fed batch process (feed process). A summary of known culture methods is described in the textbook by Chmiel (Bioprozesstechnik 1. Einführung in die Bioverfahrenstechnik [Bioprocess Technology 1. Introduction to Bioprocess Technology (Gustav Fischer Verlag, Stuttgart, 1991)) or in the textbook by Storhas (Bioreaktoren und periphere Einrichtungen [Bioreactors and Peripheral Equipment] (Vieweg Verlag, Braunschweig/Wiesbaden, 1994)).

The culture medium to be used must meet the requirements of the particular strains in a suitable manner. Descriptions of culture media for various microorganisms are contained in the handbook "Manual of Methods for General Bacteriology" of the American Society for Bacteriology (Washington D.C., USA, 1981). Sugars and carbohydrates, such as e.g. glucose, sucrose, lactose, fructose, maltose, molasses, starch and cellulose, oils and fats, such as e.g. soya oil, sunflower oil, groundnut oil and coconut fat, fatty acids, such as e.g. palmitic acid, stearic acid and linoleic acid, alcohols, such as e.g. glycerol and ethanol, and organic acids, such as e.g. acetic acid, can be used as the source of carbon. These substance can be used individually or as a mixture. Organic nitrogen-containing compounds, such as peptones, yeast extract, meat extract, malt extract, corn steep liquor, soya bean flour and urea, or inorganic compounds, such as ammonium sulphate, ammonium chloride, ammonium phosphate, ammonium carbonate and ammonium nitrate, can be used as the source of nitrogen. The sources of nitrogen can be used individually or as a mixture. Phosphoric acid, potassium dihydrogen phosphate or dipotassium hydrogen phosphate or the corresponding sodium-containing salts can be used as the source of phosphorus. The culture medium must furthermore comprise salts of metals, such as e.g. magnesium sulfate or iron sulfate, which are necessary for growth. Finally, essential growth substances, such as amino acids and vitamins, can be employed in addition to the abovementioned substances. Suitable precursors can moreover be added to the culture medium. The starting substances mentioned can be added to the culture in the form of a single batch, or can be fed in during the culture in a suitable manner.

Basic compounds, such as sodium hydroxide, potassium hydroxide, ammonia or aqueous ammonia, or acid compounds, such as phosphoric acid or sulfuric acid, can be employed in a suitable manner to control the pH. Antifoams, such as e.g. fatty acid polyglycol esters, can be employed to control the development of foam. Suitable substances having a selective action, e.g. antibiotics, can be added to the medium to maintain the stability of plasmids. To maintain aerobic conditions, oxygen or oxygen-containing gas mixtures, such as e.g. air, are introduced into the culture. The temperature of the culture is usually 25°C to 45°C, and preferably 30°C to 40°C. Culturing is continued until a maximum of L-threonine has formed. This target is usually reached within 10 hours to 160 hours.

The analysis of L-threonine can be carried out by anion exchange chromatography with subsequent ninhydrin derivatization, as described by Spackman et al. (Analytical Chemistry, 30, (1958), 1190), or it can take place by reversed phase HPLC as described by Lindroth et al. (Analytical Chemistry (1979) 51:. 1167-1174).

The following microorganism has been deposited at the Deutsche Sammlung für Mikrorganismen und Zellkulturen (DSMZ = German Collection of Microorganisms and Cell Cultures, Braunschweig, Germany) in accordance with the Budapest Treaty:
- Brevibacterium flavum strain DM368-2 pZ1thrE as DSM 12840

The plasmid pZ1thrE contains the thrE gene of Corynebacterium glutamicum ATCC13032.

The present invention is explained in more detail in the following with the aid of embodiment examples.

The isolation of plasmid DNA from Escherichia coli and all techniques of restriction, Klenow and alkaline phosphatase treatment were carried out by the method of Sambrook et al. (Molecular cloning - A laboratory manual (1989) Cold Spring Harbour Laboratory Press). The transformation of Escherichia coli was carried out by the method of Chung et al. (Proceedings of the National Academy of Sciences of the United States of America USA (1989) 86: 2172-2175).

The incubation temperature during the preparation of E. coli strains and transformants was 37°C. In the gene replacement process of Hamilton et al. (Journal of Bacteriology (1989) 171: 4617-4622) temperatures of 30°C and 44°C were used.

### Example 1

Cloning and sequencing of the thrE gene of Corynebacterium glutamicum ATCC14752

### 1. Transposon mutagenesis and mutant selection

The strain Corynebacterium glutamicum ATCC14752ΔilvA was subjected to mutagenesis with the transposon Tn5531, the sequence of which is deposited under accession number U53587 in the nucleotide databank of the National Center for Biotechnology Information (Bethesda, USA). The incorporation of a deletion in the ilvA gene of Corynebacterium glutamicum ATCC14752 was carried out with the gene exchange system described by Schäfer et al. (Gene (1994) 145: 69-73). For this, an inactivation vector pK19mobsacBΔilvA constructed by Sahm et al. (Applied and Environmental Microbiology (1999) 65: 1973 -1979) was used for the deletion. The methylase-defective Escherichia coli strain SCS110 (Jerpseth and Kretz, STRATEGIES in molecular biology 6, 22, (1993)) from Stratagene (Heidelberg, Germany) was first transformed with 200 ng of the vector pK19mobsacBΔilvA. Transformants were identified with the aid of their kanamycin resistance on LB agar plates containing 50 µg/mL kanamycin. The plasmid pK19mobsacBΔilvA was prepared from one of the transformants. By means of electroporation (Haynes et al., FEMS Microbiology Letters (1989) 61: 329-334), this inactivation plasmid was then introduced into the strain Corynebacterium glutamicum ATCC14752. Clones in which the inactivation vector was present integrated in the genome were identified with the aid of their kanamycin resistance on LBHIS agar plates containing 15 µg/mL kanamycin (Liebl et al., FEMS Microbiology Letters (1989) 65:« 299-304). To select for the excision of the vector, kanamycin-resistant clones were plated out on sucrose-containing LBG medium (LB medium with 15 g/L agar, 2% glucose and 10% sucrose). This gave colonies which have lost the vector again by a second recombination event (Jäger et al.; Journal of Bacteriology (1992) 174: 5462-5465). By transinoculation on to minimal medium plates (CGXII medium with 15 g/L Agar (Keilhauer et al., Journal of Bacteriology (1993) 175: 5595-5603)) with and without 300 mg/L L-isoleucine, or with and without 50 µg/mL kanamycin, six clones were isolated which were kanamycin-sensitive and isoleucine-auxotrophic due to excision of the vector and in which the incomplete ilvA gene (ΔilvA allele) was now present in the genome. One of these clones was designated strain ATCC14752ΔilvA and employed for the transposon mutagenesis.

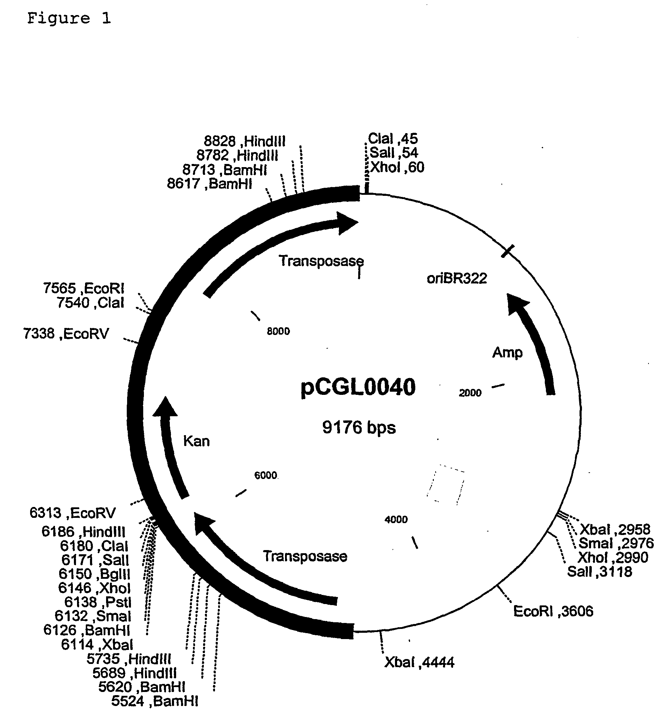

From the methylase-defective E. coli strain GM2929pCGL0040 (E. coli GM2929: Palmer et al., Gene (1994) 143: 1-12) was isolated the plasmid pCGL0040 (figure 1), which contains the combined transposon Tn5531 (Ankri et al., Journal of Bacteriology (1996) 178:« 4412-4419). The strain Corynebacterium glutamicum ATCC14752ΔilvA was transformed by means of electroporation (Haynes et al., FEMS Microbiology Letters (1989) 61: 329-334) with the plasmid pCGL0040. Clones in which the transposon Tn5531 was integrated into the genome were identified with the aid of their kanamycin resistance on LBHIS agar plates containing 15 µg/mL kanamycin (Liebl et al., FEMS Microbiology Letters (1989) 65: 299-304). 2000 clones were obtained in this manner, and were investigated for delayed growth in the presence of threonyl-threonyl-threonine. For this, all the clones were transferred individually to CGXII minimal medium agar plates with and without 2 mM threonyl-threonyl-threonine. The medium was identical to the CGXII medium described by Keilhauer et al. (Journal of Bacteriology (1993) 175: 5593-5603), but additionally contained 25 µg/mL kanamycin, 300 mg/L L-isoleucine and 15 g/L agar. The composition of the medium described by Keilhauer et al. is shown in table 1.

**Table 1**

| Composition of the CGXII medium | |
|---|---|
| Component | Concentration |
| (NH₄)₂SO₄ | 20 g/L |
| Urea | 5 g/L |
| KH₂PO₄ | 1 g/L |
| K₂HPO₄ | 1 g/L |
| MgSO₄ x 7 H₂O | 0.25 g/L |
| 3-Morpholinopropanesulfonic acid | 42 g/L |
| CaCl₂ | 10 mg/L |
| FeSO₄ x 7 H₂O | 10 mg/L |
| MnSO₄ x H₂O | 10 mg/L |
| ZnSO₄ x 7H₂O | 1 mg/L |
| CuSO₄ | 0.2 mg/L |
| NiCl₂ x 6 H₂O | 0.02 mg/L |
| Biotin | 0.2 mg/L |
| Glucose | 40 g/L |
| Protocatechuic acid | 30 mg/L |

The agar plates were incubated at 30°C and the growth was investigated after 12, 18 and 24 hours. A transposon mutant was obtained which grew in a manner comparable to the starting strain Corynebacterium glutamicum ATCC14752ΔilvA without threonyl-threonyl-threonine but showed delayed growth in the presence of 2 mM threonyl-threonyl-threonine. This was designated ATCC14752ΔilvAthrE::Tn5531.

### 2. Cloning and sequencing of the insertion site of Tn5531 in ATCC14752ΔilvAthrE::Tn5531

To clone the insertion site lying upstream of the transposon Tn5531 in the mutant described in example 1.1, the chromosomal DNA of this mutant strain was first isolated as described by Schwarzer et al. (Bio/Technology (1990) 9: 84-87) and 400 ng thereof were cleaved with the restriction endonuclease EcoRI. The complete restriction batch was ligated in the vector pUC18 (Norander et al., Gene (1983) 26: 101-106, Roche Diagnostics, (Mannheim, Germany), which was also linearized with EcoRI. The E. coli strain DH5αmcr (Grant et al., Proceedings of the National Academy of Sciences of the United States of America USA (1990) 87: 4645-4649) was transformed with the entire ligation batch by means of electroporation (Dower et al., Nucleic Acid Research (1988) 16: 6127-6145). Transformants in which the insertion sites of the transposon Tn5531 were present cloned on the vector pUC18 were identified with the aid of their carbenicillin and kanamycin resistance on LB agar plates containing 50 µg/mL carbenicillin and 25 µg/mL kanamycin. The plasmids were prepared from three of the transformants and the sizes of the cloned inserts were determined by restriction analysis. The nucleotide sequence of the insertion site on one of the plasmids with an insert approx. 5.7 kb in size was determined by the dideoxy chain termination method of Sanger et al. (Proceedings of the National Academy of Sciences of the United States of America USA (1977) 74: 5463-5467). For this, 2.2 kb of the insert were sequenced starting from the following oligonucleotide primer: 5'-CGG GTC TAC ACC GCT AGC CCA GG-3'.

For identification of the insertion site lying downstream of the transposon, the chromosomal DNA of the mutant was cleaved with the restriction endonuclease XbaI and ligated in the vector pUC18 linearized with XbaI. Further cloning was carried out as described above. The nucleotide sequence of the insertion site on one of the plasmids with an insert approx. 8.5 kb in size was determined by the dideoxy chain termination method of Sanger et al. (Proceedings of the National Academy of Sciences of the United States of America USA (1977) 74: 5463-5467). For this, 0.65 kb of the insert were sequenced starting from the following oligonucleotide primer: 5'-CGG TGC CTT ATC CAT TCA GG-3'.

The nucleotide sequences obtained were analysed and joined together with the Lasergene program package (Biocomputing Software for Windows, DNASTAR, Madison, USA)). This nucleotide sequence is reproduced as SEQ ID NO 1. The result of the analysis was identification of an open reading frame of 1467 bp in length. The corresponding gene was designated the thrE gene. The associated gene product comprises 489 amino acids and is reproduced as SEQ ID NO 2.

### Example 2

### Cloning and sequencing of the thrE gene from Corynebacterium glutamicum ATCC13032

The thrE gene was cloned in the E. coli cloning vector pUC18 (Norrander et al., Gene (1983) 26: 101-106, Roche Diagnostics, Mannheim, Germany). The cloning was carried out in two steps. The gene from Corynebacterium glutamicum ATCC13032 was first amplified by a polymerase chain reaction (PCR) by means of the following oligonucleotide primers derived from SEQ ID NO 1.
thrE-forward:
5'-CCC CTT TGA CCT GGT GTT ATT G-3'
thrE-reverse:
5'-CGG CTG CGG TTT CCT CTT-3'

The PCR reaction was carried out in 30 cycles in the presence of 200 µM deoxynucleotide triphosphates (dATP, dCTP, dGTP, dTTP), in each case 1 µM of the corresponding oligonucleotide, 100 ng chromosomal DNA from Corynebacterium glutamicum ATCC13032, 1/10 volume 10-fold reaction buffer and 2.6 units of a heat-stable Taq-/Pwo-DNA polymerase mixture (Expand High Fidelity PCR System from Roche Diagnostics, Mannheim, Germany) in a Thermocycler (PTC-100, MJ Research, Inc., Watertown, USA) under the following conditions: 94°C for 30 seconds, 58°C for 30 seconds and 72°C for 2 minutes.

The amplified fragment about 1.9 kb in size was then subsequently ligated with the aid of the SureClone Ligation Kit (Amersham Pharmacia Biotech, Uppsala, Sweden) into the SmaI cleavage site of the vector pUC18 in accordance with the manufacturer's instructions. The E. coli strain DH5αmcr (Grant et al., Proceedings of the National Academy of Sciences of the United States of America USA (1990) 87: 4645-4649) was transformed with the entire ligation batch. Transformants were identified with the aid of their carbenicillin resistance on LB agar plates containing 50 µg/mL carbenicillin. The plasmids were prepared from 8 of the transformants and checked for the presence of the 1.9 kb PCR fragment as an insert by restriction analysis.

The recombinant plasmid formed in this way is designated pUC18thrE in the following.

The nucleotide sequence of the 1.9 kb PCR fragment in plasmid pUC18thrE was determined by the dideoxy chain termination method of Sanger et al. (Proceedings of the National Academy of Sciences of the United States of America USA (1977) 74: 5463-5467). For this, the complete insert of pUC18thrE was sequenced with the aid of the following primers from Roche Diagnostics (Mannheim, Germany).
Universal primer:
5'-GTA AAA CGA CGG CCA GT-3'
Reverse primer:
5'-GGA AAC AGC TAT GAC CAT G-3'

The nucleotide sequence is reproduced as SEQ ID NO 3. The nucleotide sequence obtained was analysed with the Lasergene program package (Biocomputing Software for Windows, DNASTAR, Madison, USA). The result of the analysis was identification of an open reading frame of 1467 bp in length, which was designated the thrE gene.
This codes for a polypeptide of 489 amino acids, which is reproduced as SEQ ID NO 4.

### Example 3

### Expression of the thrE gene in Corynebacterium glutamicum

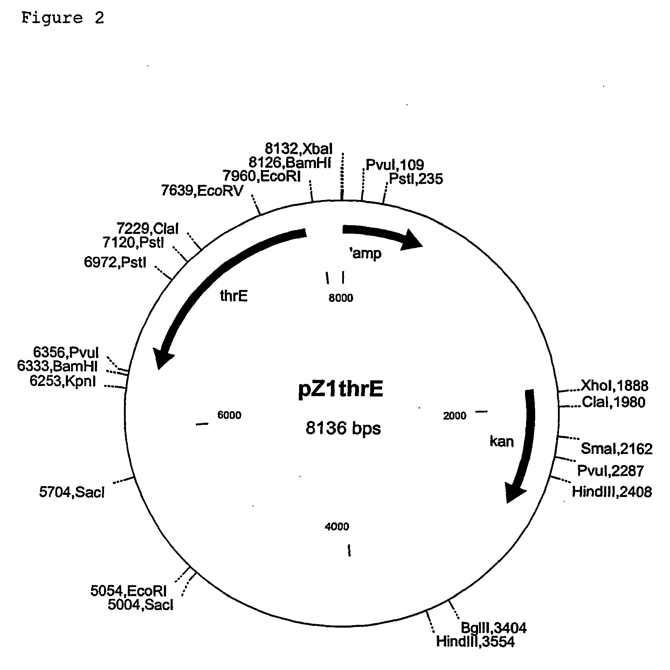

The thrE gene from Corynebacterium glutamicum ATCC13032 described under example 2 was cloned for expression in the vector pZ1 (Menkel et al., Applied and Environmental Microbiology (1989) 64: 549-554). For this, a DNA fragment 1881 bp in size which contained the thrE gene was cut out of the plasmid pUC18thrE with the restriction enzymes SacI and XbaI. The 5' and 3' ends of this fragment were treated with Klenow enzyme. The resulting DNA fragment was ligated in the vector pZ1, which was linearized with Scat and dephosphorylated beforehand. The E. coli strain DH5αmcr (Grant et al., Proceedings of the National Academy of Sciences of the United States of America USA (1990) 87: 4645-4649) was transformed with the entire ligation batch. Transformants were identified with the aid of their kanamycin resistance on LB agar plates containing 50 µg/mL kanamycin. The plasmids were prepared from 2 transformants and checked for the presence of the 1881 bp ScaI/XbaI fragment as an insert by restriction analysis. The recombinant plasmid formed in this manner was designated pZ1thrE (figure 2).

By means of electroporation (Haynes et al., FEMS Microbiology Letters (1989) 61: 329-334), the plasmid pZ1thrE was introduced into the threonine-forming strain Brevibacterium flavum DM368-2. The strain DM368-2 is described in EP-B-O 385 940 and deposited as DSM5399. Transformants were identified with the aid of their kanamycin resistance on LBHIS agar places containing 15 µg/mL kanamycin (Liebl et al., FEMS Microbiology Letters (1989) 65: 299-304). The strain Brevibacterium flavum DM368-2 pZ1thrE was formed in this manner.

### Example 4

### Construction of the expression plasmid pTrc99AthrE

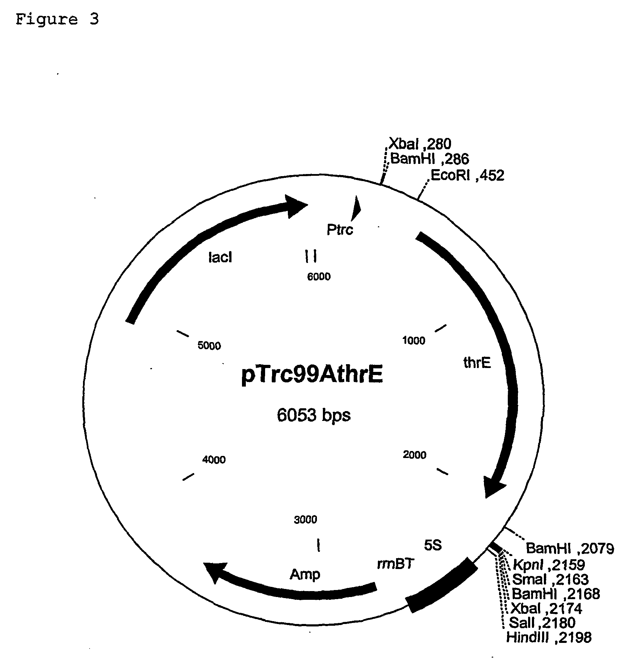

The thrE gene from Corynebacterium glutamicum ATCC13032 described under example 2 was cloned for expression in Escherichia coli in the vector pTrc99A, which was obtained from Pharmacia Biotech (Uppsala, Sweden), for expression in Escherichia coli. For this, the plasmid pUC18thrE was cleaved with the enzyme SalI and the projecting 3' ends were treated with Klenow enzyme. After restriction with the enzyme KpnI, the cleavage batch was separated in 0.8% agarose gel and the thrE fragment 1.9 kbp in size was isolated with the aid of the QIAquick Gel Extraction Kit (QIAGEN, Hilden, Germany). The vector pTrc99A was cleaved with the enzyme EcoRI, the 3' ends were treated with Klenow enzyme, cleaved with the enzyme KpnI and ligated with the thrE fragment isolated. The ligation batch was transformed in the E. coli strain DH5α. Selection of cells carrying pTrc99A was carried out on LB agar (Lennox, Virology 1:190 (1955)), to which 50 µg/ml ampicillin had been added. Successful cloning of the thrE gene could be demonstrated after plasmid DNA isolation and control cleavage with XbaI, BamHI. EcoRI, HindIII and SspI. The plasmid was designated pTrc99AthrE (figure 3).

### Example 5

### Preparation of L-threonine with the strain MG442/pTrc99AthrE

The L-threonine-producing E. coli strain MG442 is described in the patent specification US-A- 4,278,765 and deposited at the Russian National Collection of Industrial Microorganisms (VKPM, Moscow, Russia) as CMIM B-1628.

The strain MG442 was transformed with the plasmid pTrc99AthrE and plasmid-carrying cells were selected on LB agar with 50 µg/ml ampicillin. Selected individual colonies were then multiplied further on minimal medium with the following composition: 3.5 g/l Na₂HPO₄*2H₂O, 1.5 g/l KH₂PO₄, 1 g/l NH₄Cl, 0.1 g/l MgSO₄*7H₂O, 2 g/l glucose, 20 g/l agar, 50 mg/l ampicillin. The formation of L-threonine was checked in batch cultures of 10 ml contained in 100 ml conical flasks. For this, 10 ml preculture medium of the following composition: 2 g/l yeast extract, 10 g/l (NH₄)₂SO₄, 1 g/l KH₂PO₄, 0.5 g/l MgSO₄*7H₂O, 15 g/l CaCO₃, 20 g/l glucose, 50 mg/l ampicillin were inoculated and incubated for 16 hours at 37°C and 180 rpm on an ESR incubator from Kühner AG (Birsfelden, Switzerland). 250 µl portions of this preculture were transinoculated into 10 ml of production medium (25 g/l (NH₄)₂SO₄, 2 g/l KH₂PO₄, 1 g/l MgSO₄*7H₂O, 0.03 g/l FeSO₄*7H₂O, 0.018 g/l MnSO₄*1H₂O, 30 g/l CaCO₃, 20 g/l glucose) and the mixtures were incubated for 48 hours at 37°C. For induction of the expression of the thrE gene, 200 mg/l isopropyl β-D-thiogalactoside (IPTG) were added in parallel batches. After the incubation, the optical density (OD) of the culture suspension was determined with an LP2W photometer from Dr. Lange (Berlin, Germany) at a measurement wavelength of 660 nm.

The concentration of L-threonine formed was then determined in the sterile-filtered culture supernatant with an amino acid analyser from Eppendorf-BioTronik (Hamburg, Germany) by ion exchange chromatography and post-column reaction with ninhydrin detection.

The result of the experiment is shown in table 2.

**Table 2**

| Strain | Additives | OD | L-Threonine g/l |
|---|---|---|---|
| MG442 | - | 5.6 | 1.38 |
| MG442/pTrc99AthrE | - | 4.6 | 1.65 |
| MG442/pTrc99AthrE | IPTG | 3.6 | 3.5 |

### Example 6

### Preparation of L-threonine with the strain B-3996kurΔtdh/pVIC40, pMW218thrE

The L-threonine-producing E. coli strain B-3996 is described in US-A- 5,175,107 and deposited at the Russian National Collection for Industrial Microorganisms (VKPM, Moscow, Russia).

### 6.1 Cloning of the thrE gene in the plasmid vector pMW218

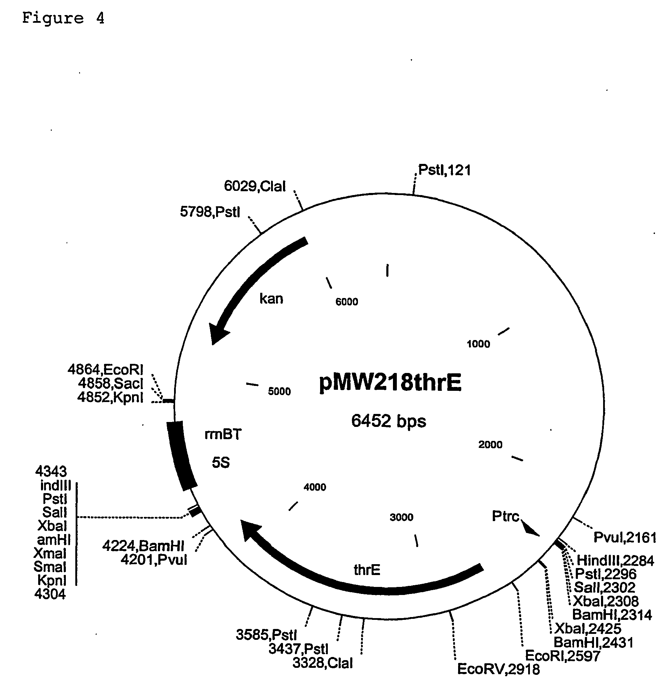

The plasmid pTrc99AthrE described under example 4 was cleaved with the enzyme SspI, the cleavage batch was separated in 0.8% agarose gel and the DNA fragment 2.5 kbp in size, which contained the trc promoter region and the rRNA terminator region in addition to the thrE gene, was isolated with the aid of the "QIAquick Gel Extraction Kit" (QIAGEN, Hilden, Germany). The plasmid pMW218 (Nippon Gene, Toyama, Japan) was cleaved with the enzyme SmaI and ligated with the thrE fragment. The E. coli strain DH5α was transformed with the ligation batch and pMW218-carrying cells were selected by plating out on LB agar, to which 20 µg/ml kanamycin are added. Successful cloning of the thrE gene could be demonstrated after plasmid DNA isolation and control cleavage with HindIII and ClaI. The plasmid was designated pMW218thrE (figure 4).

### 6.2 Preparation of the strain B-3996kurΔtdh/pVIC40, pMW218thrE

After culture in antibiotic-free complete medium for approximately ten generations, a derivative of strain B-3996 which no longer contained the plasmid pVIC40 was isolated. The strain formed was streptomycin-sensitive and was designated B-3996kur.

The method described by Hamilton et al. (Journal of Bacteriology (1989) 171: 4617-4622), which is based on the use of the plasmid pMAK705 with a temperature-sensitive replicon, was used for incorporation of a deletion into the tdh gene. The plasmid pDR121 (Ravnikar and Somerville, Journal of Bacteriology (1987) 169: 4716-4721) contains a DNA fragment from E. coli 3.7 kilo-base pairs (kbp) in size, on which the tdh gene is coded. To generate a deletion of the tdh gene region, pDR121 was cleaved with the restriction enzymes ClaI and EcoRV and the DNA fragment 5 kbp in size isolated was ligated, after treatment with Klenow enzyme. The ligation batch was transformed in the E. coli strain DH5α and plasmid-carrying cells were selected on LB agar, to which 50 µg/ml ampicillin are added.

Successful deletion of the tdh gene could be demonstrated after plasmid DNA isolation and control cleavage with EcoRI. The EcoRI fragment 1.7 kbp in size was isolated, and ligated with the plasmid pMAK705, which was partly digested with EcoRI. The ligation batch was transformed in DH5α and plasmid-carrying cells were selected on LB agar, to which 20 µg/ml chloramphenicol were added. Successful cloning was demonstrated after isolation of the plasmid DNA and cleavage with EcoRI. The pMAK705 derivative formed was designated pDM32.

For the gene replacement, B-3996kur was transformed with the plasmid pDM32. The replacement of the chromosomal tdh gene with the plasmid-coded deletion construct was carried out by the selection process described by Hamilton et al. and was verified by standard PCR methods (Innis et al. (1990), PCR Protocols. A Guide to Methods and Applications, Academic Press) with the following oligonucleotide primers:
Tdh1: 5'-TCGCGACCTATAAGTTTGGG-3'
Tdh2: 5'-AATACCAGCCCTTGTTCGTG-3'

The strain formed was tested for kanamycin sensitivity and was designated B-3996kurΔtdh.

B-3996kurΔtdh was transformed with the plasmid pVIC40 isolated from B-3996 and plasmid-carrying cells were selected on LB agar supplemented with 20 µg/ml streptomycin. A selected individual colony was designated B-3996kurΔtdh/pVIC40 and transformed with the plasmid pMW218thrE. Selection is carried out on LB-agar to which 20 µg/ml streptomycin and 50 µg/ml kanamycin are added. The strain formed in this way was designated B-3996kurΔtdh/pVIC40, pMW218thrE.

### 6.3 Preparation of L-threonine

The preparation of L-threonine by the strains B-3996kurΔtdh/pVIC40 and B-3996kurΔtdh/pVIC40, pMW218thrE was tested as described in example 5. The minimal medium, the preculture medium and the production medium were supplemented with 20 µg/ml streptomycin for B-3996kurΔtdh/pVIC40 and with 20 µg/ml streptomycin and 50 µg/ml kanamycin for B-3996kurΔtdh/pVIC40, pMW218thrE.

The result of the experiment is summarized in table 3.

**Table 3**

| Strain | OD (660 nm) | L-Threonine g/l |
|---|---|---|
| B-3996kurΔtdh/pVIC40 | 4.7 | 6.26 |
| B-3996kurΔtdh/pVIC40, pMW218thrE | 4.8 | 7.57 |

The following figures are attached:
- Figure 1: Map of the plasmid pCGL0040 containing the transposon Tn5531. The transposon is identified as the non-shaded arrow.
- Figure 2: Map of the plasmid pZ1thrE containing the thrE gene.
- Figure 3: Map of the plasmid pTrc99AthrE containing the thrE gene.
- Figure 4: Map of the plasmid pMW218thrE containing the thrE gene

The length data are to be understood as approx. data. The abbreviations and designations used have the following meaning:
- Amp: Ampicillin resistance gene
- Kan: Kanamycin resistance gene
- 'amp: 3' part of the ampicillin resistance gene
- oriBR322:Replication region of the plasmid pBR322
- lacI: Gene for the repressor protein of the trc promoter
- Ptrc: trc promoter region, IPTG-inducible
- 5S: 5S rRNA region
- rrnBT: rRNA terminator region

The abbreviations for the restriction enzymes have the following meaning
- BamHI: Restriction endonuclease from Bacillus amyloliquefaciens
- BglII: Restriction endonuclease from Bacillus globigii
- ClaI: Restriction endonuclease from Caryphanon latum
- EcoRI: Restriction endonuclease from Escherichia coli
- EcoRV: Restriction endonuclease from Escherichia coli
- HindIII: Restriction endonuclease from Haemophilus influenzae
- KpnI: Restriction endonuclease from Klebsiella pneumoniae
- PstI: Restriction endonuclease from Providencia stuartii
- PvuI: Restriction endonuclease from Proteus vulgaris
- SacI: Restriction endonuclease from Streptomyces achromogenes
- SalI: Restriction endonuclease from Streptomyces albus
- SmaI: Restriction endonuclease from Serratia marcescens
- XbaI: Restriction endonuclease from Xanthomonas badrii
- XhoI: Restriction endonuclease from Xanthomonas holcicola

## Claims

1. A process for the fermentative preparation of L-threonine, which comprises employing Enterobacteriaceae bacteria, in which the thrE nucleotide sequence of coryneform bacteria which codes for the threonine export protein is over-expressed.

2. A process as claimed in claim 1, wherein the microorganisms of the family Enterobacteriaceae are from the genus Escherichia and Serratia.

3. A process as claimed in claim 2, wherein the microorganisms are of the species Escherichia coli.

4. A process as claimed in claim 1, wherein the thrABC operon which codes for aspartate kinase, homoserine dehydrogenase, homoserine kinase and threonine synthase is overexpressed at the same time.

5. A process as claimed in claim 1, wherein the pyc gene which codes for pyruvate carboxylase is overexpressed at the same time.

6. A process as claimed in claim 1, wherein the pps gene which codes for phosphoenol pyruvate synthase is overexpressed at the same time.

7. A process as claimed in claim 1, wherein the ppc gene which codes for phosphoenol pyruvate carboxylase is overexpressed at the same time.

8. A process as claimed in claim 1, wherein the genes pntA and pntB which code for transhydrogenase are overexpressed at the same time.

9. A process as claimed in claim 1, wherein a strain transformed with a plasmid vector is employed and the plasmid vector carries the thrE nucleotide sequence of coryneform bacteria which codes for the threonine export protein.

10. A process as claimed in claim 1, wherein the expression of the thrE gene is induced, in particular with isopropyl β-D-thiogalactoside.

11. A process as claimed in claim 1, wherein the gdhA gene which codes for glutamate dehydrogenase is overexpressed at the same time.

12. A process as claimed in claim 1, wherein the rhtB gene which imparts homoserine resistance is overexpressed at the same time.

13. A process for the preparation of L-threonine according to claim 1, which comprises carrying out the following steps:
a) fermentation of microorganisms of the family Enterobacteriaceae in which at least the thrE gene of coryneform bacteria is over-expressed,
b) concentration of the L-threonine in the medium or in the cells of the microorganisms of the family Enterobacteriaceae, and
c) isolation of the L-threonine.

## Patentansprüche

1. Verfahren zur fermentativen Herstellung von L-Threonin, **dadurch gekennzeichnet, daß** man Enterobacteriaceae Bakterien einsetzt, in denen man die für das Threonin-Exportprotein codierende thrE-Nukleotidsequenz coryneformer Bakterien überexprimiert.

2. Verfahren gemäß Anspruch 1, **dadurch gekennzeichnet, daß** die Mikroorganismen der Familie Enterobacteriaceae aus der Gattung Escherichia und Serratia sind.

3. Verfahren gemäß Anspruch 2, **dadurch gekennzeichnet, daß** die Mikroorganismen aus der Art Escherichia coli sind.

4. Verfahren gemäß Anspruch 1, **dadurch gekennzeichnet, daß** man gleichzeitig das für die Aspartatkinase, die Homoserin-Dehydrogenase, die Homoserinkinase und die Threoninsynthase Codierende thrABC-Operon überexprimiert.

5. Verfahren gemäß Anspruch 1, **dadurch gekennzeichnet, daß** man gleichzeitig das für die Pyruvat-Carboxylase Codierende pyc-Gen überexprimiert.

6. Verfahren gemäß Anspruch 1, **dadurch gekennzeichnet, daß** man gleichzeitig das für die Phosphoenolpyruvat-Synthase Codierende pps-Gen überexprimiert.

7. Verfahren gemäß Anspruch 1, **dadurch gekennzeichnet, daß** man gleichzeitig das für die Phosphoenolpyruvat-Carboxylase Codierende ppc-Gen überexprimiert.

8. Verfahren gemäß Anspruch 1, **dadurch gekennzeichnet, daß** man gleichzeitig die für die Transhydrogenase Codierenden Gene pntA und pntB überexprimiert.

9. Verfahren gemäß Anspruch 1, **dadurch gekennzeichnet, daß** man einen mit einem Plasmidvektor transformierten Stamm einsetzt und der Plasmidvektor die für das Threonin-Exportprotein codierende thrE-Nukleotidsequenz coryneformer Bakterien trägt.

10. Verfahren gemäß Anspruch 1, **dadurch gekennzeichnet, daß** man die Expression des thrE-Gens induziert, insbesondere mit Isopropyl-β-D-thiogalaktosid.

11. Verfahren gemäß Anspruch 1, **dadurch gekennzeichnet, daß** man gleichzeitig das für die Glutamat-Dehydrogenase Codierende gdhA-Gen überexprimiert.

12. Verfahren gemäß Anspruch 1, **dadurch gekennzeichnet, daß** man gleichzeitig das Homoserinresistenz vermittelnde rhtB-Gen überexprimiert.

13. Verfahren zur Herstellung von L-Threonin gemäß Anspruch 1, **dadurch gekennzeichnet, daß** man folgende Schritte durchführt:
a) Fermentation von Mikroorganismen der Familie Enterobacteriaceae in denen zumindest das thrE-Gen coryneformer Bakterien überexprimiert wird,
b) Anreicherung des L-Threonins im Medium oder in den Zellen der Mikroorganismen der Familie Enterobacteriaceae, und
c) Isolierung des L-Threonins.

## Revendications

1. Procédé de préparation par fermentation de L-thréonine, qui comprend l'utilisation de bactéries d'Enterobacteriaceae dans lesquelles la séquence nucléotidique thrE de bactéries coryneformes qui code pour la protéine d'exportation de la thréonine est surexprimée.

2. Procédé selon la revendication 1, **caractérisé en ce que** les microorganismes de la famille des Enterobacteriaceae appartiennent aux genres Escherichia et Serratia.

3. Procédé selon la revendication 2, **caractérisé en ce que** les microorganismes sont de l'espèce Escherichia coli.

4. Procédé selon la revendication 1, **caractérisé en ce que** l'opéron thrABC qui code pour l'aspartate kinase, l'homosérine déshydrogénase, l'homosérine kinase et la thréonine synthase est surexprimé en même temps.

5. Procédé selon la revendication 1, **caractérisé en ce que** le gène pyc qui code pour la pyruvate carboxylase est surexprimé en même temps.

6. Procédé selon la revendication 1, **caractérisé en ce que** le gène pps qui code pour la phosphoénol pyruvate synthase est surexprimé en même temps.

7. Procédé selon la revendication 1, **caractérisé en ce que** le gène ppc qui code pour la phosphoénol pyruvate carboxylase est surexprimé en même temps.

8. Procédé selon la revendication 1, **caractérisé en ce que** les gènes pntA et pntB qui codent pour la transhydrogénase sont surexprimés en même temps.

9. Procédé selon la revendication 1, **caractérisé en ce qu'**une souche transformée par un vecteur plasmidique est utilisée et **en ce que** le vecteur plasmidique porte la séquence nucléotidique thrE de bactéries coryneformes qui code pour la protéine d'exportation de la thréonine.

10. Procédé selon la revendication 1, **caractérisé en ce que** l'expression du gène thrE est induite, en particulier par l'isopropyl β-D-thiogalactoside.

11. Procédé selon la revendication 1, **caractérisé en ce que** le gène gdhA qui code pour la glutamate déshydrogénase est surexprimé en même temps.

12. Procédé selon la revendication 1, **caractérisé en ce que** le gène rhtB qui confère une résistance à l'homosérine est surexprimé en même temps.

13. Procédé de préparation de L-thréonine selon la revendication 1, qui comprend l'exécution des étapes suivantes :
a) la fermentation de microorganismes de la famille des Enterobacteriaceae dans lesquels au moins le gène thrE de bactéries coryneformes est surexprimé,
b) la concentration de la L-thréonine dans le milieu ou dans les cellules des microorganismes de la famille des Enterobacteriaceae, et
c) l'isolement de la L-thréonine.


### SEQUENCE PROTOCOL

<110> Degussa AG
<120> Process for the fermentative preparation of L-threonine.
<130> 000225 BT
<140>
   <141>
<160> 4
<170> Patent In Ver. 2.1
<210> 1
   <211> 2817
   <212> DNA
   <213> Corynebacterium glutamicum ATCC14752
<220>
   <221> CDS
   <222> (398)..(1864)
   <223> thrE gene
<400> 1
   aatgaaataa tcccctcacc aactggcgac attcaaacac cgtttcattt ccaaacatcg 60
   agccaaggga aaagaaagcc cctaagcccc gtgttattaa atggagactc tttggagacc 120
   tcaagccaaa aaggggcatt ttcattaaga aaatacccct ttgacctggt gttattgagc 180
   tggagaagag acttgaactc tcaacctacg cattacaagt gcgttgcgct gccaattgcg 240
   ccactccagc accgcagatg ctgatgatca acaactacga atacgtatct tagcgtatgt 300
   gtacatcaca atggaattcg gggctagagt atctggtgaa ccgtgcataa acgacctgtg 360
   attggactct ttttccttgc aaaatgtttt ccagcgg atg ttg agt ttt gcg acc  415
                                          Met Leu Ser Phe Ala Thr
                                           1               5
   ctt cgt ggc cgc att tca aca gtt gac gct gca aaa gcc gca cct ccg   463
   Leu Arg Gly Arg Ile Ser Thr Val Asp Ala Ala Lys Ala Ala Pro Pro
               10                  15                  20
   cca tcg cca cta gcc ccg att gat ctc act gac cat agt caa gtg gcc   511
   Pro Ser Pro Leu Ala Pro Ile Asp Leu Thr Asp His Ser Gln Val Ala
           25                  30                  35
   ggt gtg atg aat ttg gct gcg aga att ggc gat att ttg ctt tct tca   559
   Gly Val Met Asn Leu Ala Ala Arg Ile Gly Asp Ile Leu Leu Ser Ser
       40                  45                  50
   ggt acg tca aac agt gat acc aag gtg caa gtt cga gcg gtg acc tct   607
   Gly Thr Ser Asn Ser Asp Thr Lys Val Gln Val Arg Ala Val Thr Ser
   55                  60                  65                  70
   gcg tat ggc ctg tac tat acg cat gtg gat atc acg ttg aat acg atc   655
   Ala Tyr Gly Leu Tyr Tyr Thr His Val Asp Ile Thr Leu Asn Thr Ile
                   75                  80                  85
   acc atc ttc acc aac atc ggt gtg gag agg aag atg ccg gtc aac gtg   703
   Thr Ile Phe Thr Asn Ile Gly Val Glu Arg Lys Met Pro Val Asn Val
               90                  95                  100
   ttt cat gtt gtg ggc aag ttg gac acc aac ttc tcc aaa ctg tct gag     751
   Phe His Val Val Gly Lys Leu Asp Thr Asn Phe Ser Lys Leu Ser Glu
           105                 110                 115
   gtt gac cgt ttg atc cgt tcc att cag gct ggt gct acc ccg cct gag     799
   Val Asp Arg Leu Ile Arg Ser Ile Gln Ala Gly Ala Thr Pro Pro Glu
       120                 125                 130
   gtt gcc gag aaa att ctg gac gag ttg gag caa tcg cct gcg tct tat     847
   Val Ala Glu Lys Ile Leu Asp Glu Leu Glu Gln Ser Pro Ala Ser Tyr
   135                 140                 145                 150
   ggt ttc cct gtt gcg ttg ctt ggc tgg gca atg atg ggt ggc gct gtt     895
   Gly Phe Pro Val Ala Leu Leu Gly Trp Ala Met Met Gly Gly Ala Val
                   155                 160                 165
   gct gtg ctg ttg ggt ggt gga tgg cag gtt tcc cta att gct ttt att     943
   Ala Val Leu Leu Gly Gly Gly Trp Gln Val Ser Leu Ile Ala Phe Ile
               170                 175                 180
   acc gcg ttc acg atc att gcc acg acg tca ttt ttg gga aag aag ggt     991
   Thr Ala Phe Thr Ile Ile Ala Thr Thr Ser Phe Leu Gly Lys Lys Gly
           185                 190                 195
   ttg cct act ttc ttc caa aat gtt gtt ggt ggt ttt att gcc acg ctg     1039
   Leu Pro Thr Phe Phe Gln Asn Val Val Gly Gly Phe Ile Ala Thr Leu
       200                 205                 210
   cct gca tcg att gct tat tct ttg gcg ttg caa ttt ggt ctt gag atc     1087
   Pro Ala Ser Ile Ala Tyr Ser Leu Ala Leu Gln Phe Gly Leu Glu Ile
   215                 220                 225                 230
   aaa ccg agc cag atc atc gca tct gga att gtt gtg ctg ttg gca ggt     1135
   Lys Pro Ser Gln Ile Ile Ala Ser Gly Ile Val Val Leu Leu Ala Gly
                   235                 240                 245
   ttg aca ctt gtg caa tct ctg cag gac ggc atc acg ggc gct ccg gtg     1183
   Leu Thr Leu Val Gln Ser Leu Gln Asp Gly Ile Thr Gly Ala Pro Val
               250                 255                 260
   aca gca agt gca cga ttt ttt gaa aca ctc ctg ttt acc ggc ggc att     1231
   Thr Ala Ser Ala Arg Phe Phe Glu Thr Leu Leu Phe Thr Gly Gly Ile
           265                 270                 275
   gtt gct ggc gtg ggt ttg ggc att cag ctt tct gaa atc ttg cat gtc     1279
   Val Ala Gly Val Gly Leu Gly Ile Gln Leu Ser Glu Ile Leu His Val
       280                 285                 290
   atg ttg cct gcc atg gag tcc gct gca gca cct aat tat tcg tct aca     1327
   Met Leu Pro Ala Met Glu Ser Ala Ala Ala Pro Asn Tyr Ser Ser Thr
   295                 300                 305                 310
   ttc gcc cgc att atc gct ggt ggc gtc acc gca gcg gcc ttc gca gtg     1375
   Phe Ala Arg Ile Ile Ala Gly Gly Val Thr Ala Ala Ala Phe Ala Val
                   315                 320                 325
   ggt tgt tac gcg gag tgg tcc tcg gtg att att gcg ggg ctt act gcg     1423
   Gly Cys Tyr Ala Glu Trp Ser Ser Val Ile Ile Ala Gly Leu Thr Ala
               330                 335                 340
   ctg atg ggt tct gcg ttt tat tac ctc ttc gtt gtt tat tta ggc ccc   1471
   Leu Met Gly Ser Ala Phe Tyr Tyr Leu Phe Val Val Tyr Leu Gly Pro
           345                 350                 355
   gtc tct gcc gct gcg att gct gca aca gca gtt ggt ttc act ggt ggt   1519
   Val Ser Ala Ala Ala Ile Ala Ala Thr Ala Val Gly Phe Thr Gly Gly
       360                 365                 370
   ttg ctt gcc cgt cga ttc ttg att cca ccg ttg att gtg gcg att gcc   1567
   Leu Leu Ala Arg Arg Phe Leu Ile Pro Pro Leu Ile Val Ala Ile Ala
   375                 380                 385                 390
   ggc atc aca cca atg ctt cca ggt cta gca att tac cgc gga atg tac   1615
   Gly Ile Thr Pro Met Leu Pro Gly Leu Ala Ile Tyr Arg Gly Met Tyr
                   395                 400                 405
   gcc acc ttg aat gat caa aca ctc atg ggt ttc acc aac att gcg gtt   1663
   Ala Thr Leu Asn Asp Gln Thr Leu Met Gly Phe Thr Asn Ile Ala Val
               410                 415                 420
   gct tta gcc act gct tca tca ctt gcc gct ggc gtg gtt ttg ggt gag   1711
   Ala Leu Ala Thr Ala Ser Ser Leu Ala Ala Gly Val Val Leu Gly Glu
           425                 430                 435
   tgg att gcc cgc agg cta cgt cgt cca cca cgc ttc aac cca tac cgt   1759
   Trp Ile Ala Arg Arg Leu Arg Arg Pro Pro Arg Phe Asn Pro Tyr Arg
       440                 445                 450
   gca ttt acc aag gcg aat gag ttc tcc ttc cag gag gaa gct gag cag   1807
   Ala Phe Thr Lys Ala Asn Glu Phe Ser Phe Gln Glu Glu Ala Glu Gln
   455                 460                 465                 470
   aat cag cgc cgg cag aga aaa cgt cca aag act aat caa aga ttc ggt   1855
   Asn Gln Arg Arg Gln Arg Lys Arg Pro Lys Thr Asn Gln Arg Phe Gly
                   475                 480                 485
   aat aaa agg taaaaatcaa cctgcttagg cgtctttcgc ttaaatagcg          1904
   Asn Lys Arg
   tagaatatcg ggtcgatcgc ttttaaacac tcaggaggat ccttgccggc caaaatcacg 1964
   gacactcgtc ccaccccaga atcccttcac gctgttgaag aggaaaccgc agccggtgcc 2024
   cgcaggattg ttgccaccta ttctaaggac ttcttcgacg gcgtcacttt gatgtgcatg 2084
   ctcggcgttg aacctcaggg cctgcgttac accaaggtcg cttctgaaca cgaggaagct 2144
   cagccaaaga aggctacaaa gcggactcgt aaggcaccag ctaagaaggc tgctgctaag 2204
   aaaacgacca agaagaccac taagaaaact actaaaaaga ccaccgcaaa gaagaccaca 2264
   aagaagtctt aagccggatc ttatatggat gattccaata gctttgtagt tgttgctaac 2324
   cgtctgccag tggatatgac tgtccaccca gatggtagct atagcatctc ccccagcccc 2384
   ggtggccttg tcacggggct ttcccccgtt ctggaacaac atcgtggatg ttgggtcgga 2444
   tggcctggaa ctgtagatgt tgcacccgaa ccatttcgaa cagatacggg tgttttgctg 2504
   caccctgttg tcctcactgc aagtgactat gaaggcttct acgagggctt ttcaaacgca 2564
   acgctgtggc ctcttttcca cgatttgatt gttactccgg tgtacaacac cgattggtgg 2624
   catgcgtttc gggaagtaaa cctcaagttc gctgaagccg tgagccaagt ggcggcacac 2684
   ggtgccactg tgtgggtgca ggactatcag ctgttgctgg ttcctggcat tttgcgccag 2744
   atgcgccctg atttgaagat cggtttcttc ctccacattc ccttcccttc ccctgatctg 2804
   ttccgtcagc tgc                                                    2817
<210> 2
   <211> 489
   <212> PRT
   <213> Corynebacterium glutamicum ATCC14752
<400> 2
   Met Leu Ser Phe Ala Thr Leu Arg Gly Arg Ile Ser Thr Val Asp Ala
   1               5                   10                  15
   Ala Lys Ala Ala Pro Pro Pro Ser Pro Leu Ala Pro Ile Asp Leu Thr
               20                  25                  30
   Asp His Ser Gln Val Ala Gly Val Met Asn Leu Ala Ala Arg Ile Gly
           35                  40                  45
   Asp Ile Leu Leu Ser Ser Gly Thr Ser Asn Ser Asp Thr Lys Val Gln
       50                  55                  60
   Val Arg Ala Val Thr Ser Ala Tyr Gly Leu Tyr Tyr Thr His Val Asp
   65                  70                  75                  80
   Ile Thr Leu Asn Thr Ile Thr Ile Phe Thr Asn Ile Gly Val Glu Arg
                   85                  90                  95
   Lys Met Pro Val Asn Val Phe His Val Val Gly Lys Leu Asp Thr Asn
               100                 105                 110
   Phe Ser Lys Leu Ser Glu Val Asp Arg Leu Ile Arg Ser Ile Gln Ala
           115                 120                 125
   Gly Ala Thr Pro Pro Glu Val Ala Glu Lys Ile Leu Asp Glu Leu Glu
       130                 135                 140
   Gln Ser Pro Ala Ser Tyr Gly Phe Pro Val Ala Leu Leu Gly Trp Ala
   145                 150                 155                 160
   Met Met Gly Gly Ala Val Ala Val Leu Leu Gly Gly Gly Trp Gln Val
                   165                 170                 175
   Ser Leu Ile Ala Phe Ile Thr Ala Phe Thr Ile Ile Ala Thr Thr Ser
               180                 185                 190
   Phe Leu Gly Lys Lys Gly Leu Pro Thr Phe Phe Gln Asn Val Val Gly
           195                 200                 205
   Gly Phe Ile Ala Thr Leu Pro Ala Ser Ile Ala Tyr Ser Leu Ala Leu
       210                 215                 220
   Gln Phe Gly Leu Glu Ile Lys Pro Ser Gln Ile Ile Ala Ser Gly Ile
   225                 230                 235                 240
   Val Val Leu Leu Ala Gly Leu Thr Leu Val Gln Ser Leu Gln Asp Gly
                   245                 250                 255
   Ile Thr Gly Ala Pro Val Thr Ala Ser Ala Arg Phe Phe Glu Thr Leu
               260                 265                 270
   Leu Phe Thr Gly Gly Ile Val Ala Gly Val Gly Leu Gly Ile Gln Leu
           275                 280                 285
   Ser Glu Ile Leu His Val Met Leu Pro Ala Met Glu Ser Ala Ala Ala
       290                 295                 300
   Pro Asn Tyr Ser Ser Thr Phe Ala Arg Ile Ile Ala Gly Gly Val Thr
   305                 310                 315                 320
   Ala Ala Ala Phe Ala Val Gly Cys Tyr Ala Glu Trp Ser Ser Val Ile
                   325                 330                 335
   Ile Ala Gly Leu Thr Ala Leu Met Gly Ser Ala Phe Tyr Tyr Leu Phe
               340                 345                 350
   Val Val Tyr Leu Gly Pro Val Ser Ala Ala Ala Ile Ala Ala Thr Ala
           355                 360                 365
   Val Gly Phe Thr Gly Gly Leu Leu Ala Arg Arg Phe Leu Ile Pro Pro
       370                 375                 380
   Leu Ile Val Ala Ile Ala Gly Ile Thr Pro Met Leu Pro Gly Leu Ala
   385                 390                 395                 400
   Ile Tyr Arg Gly Met Tyr Ala Thr Leu Asn Asp Gln Thr Leu Met Gly
                   405                 410                 415
   Phe Thr Asn Ile Ala Val Ala Leu Ala Thr Ala Ser Ser Leu Ala Ala
               420                 425                 430
   Gly Val Val Leu Gly Glu Trp Ile Ala Arg Arg Leu Arg Arg Pro Pro
           435                 440                 445
   Arg Phe Asn Pro Tyr Arg Ala Phe Thr Lys Ala Asn Glu Phe Ser Phe
       450                 455                 460
   Gln Glu Glu Ala Glu Gln Asn Gln Arg Arg Gln Arg Lys Arg Pro Lys
   465                 470                 475                 480
   Thr Asn Gln Arg Phe Gly Asn Lys Arg
                   485
<210> 3
   <211> 1909
   <212> DNA
   <213> Corynebacterium glutamicum ATCC13032
<220>
   <221> CDS
   <222> (280)..(1746)
   <223> thrE gene
<400> 3
   agcttgcatg cctgcaggtc gactctagag gatccccccc ctttgacctg gtgttattga 60
   gctggagaag agacttgaac tctcaaccta cgcattacaa gtgcgttgcg ctgccaattg 120
   cgccactcca gcaccgcaga tgctgatgat caacaactac gaatacgtat cttagcgtat 180
   gtgtacatca caatggaatt cggggctaga gtatctggtg aaccgtgcat aaacgacctg 240
   tgattggact cttttccttt gcaaaatgtt ttccagcgg atg ttg agt ttt gcg    294
                                             Met Leu Ser Phe Ala
                                              1               5
   acc ctt cgt ggc cgc att tca aca gtt gac gct gca aaa gcc gca cct    342
   Thr Leu Arg Gly Arg Ile Ser Thr Val Asp Ala Ala Lys Ala Ala Pro
                    10                  15                  20
   ccg cca tcg cca cta gcc ccg att gat ctc act gac cat agt caa gtg    390
   Pro Pro Ser Pro Leu Ala Pro Ile Asp Leu Thr Asp His Ser Gln Val
                25                  30                  35
   gcc ggt gtg atg aat ttg gct gcg aga att ggc gat att ttg ctt tct    438
   Ala Gly Val Met Asn Leu Ala Ala Arg Ile Gly Asp Ile Leu Leu Ser
            40                  45                  50
   tca ggt acg tca aat agt gac acc aag gta caa gtt cga gca gtg acc    486
   Ser Gly Thr Ser Asn Ser Asp Thr Lys Val Gln Val Arg Ala Val Thr
        55                  60                  65
   tct gcg tac ggt ttg tac tac acg cac gtg gat atc acg ttg aat acg    534
   Ser Ala Tyr Gly Leu Tyr Tyr Thr His Val Asp Ile Thr Leu Asn Thr
    70                  75                  80                  85
   atc acc atc ttc acc aac atc ggt gtg gag agg aag atg ccg gtc aac    582
   Ile Thr Ile Phe Thr Asn Ile Gly Val Glu Arg Lys Met Pro Val Asn
                    90                  95                 100
   gtg ttt cat gtt gta ggc aag ttg gac acc aac ttc tcc aaa ctg tct    630
   Val Phe His Val Val Gly Lys Leu Asp Thr Asn Phe Ser Lys Leu Ser
               105                 110                 115
   gag gtt gac cgt ttg atc cgt tcc att cag gct ggt gcg acc ccg cct    678
   Glu Val Asp Arg Leu Ile Arg Ser Ile Gln Ala Gly Ala Thr Pro Pro
           120                 125                 130
   gag gtt gcc gag aaa atc ctg gac gag ttg gag caa tcc cct gcg tct    726
   Glu Val Ala Glu Lys Ile Leu Asp Glu Leu Glu Gln Ser Pro Ala Ser
       135                 140                 145
   tat ggt ttc cct gtt gcg ttg ctt ggc tgg gca atg atg ggt ggt gct    774
   Tyr Gly Phe Pro Val Ala Leu Leu Gly Trp Ala Met Met Gly Gly Ala
   150                 155                 160                 165
   gtt gct gtg ctg ttg ggt ggt gga tgg cag gtt tcc cta att gct ttt    822
   Val Ala Val Leu Leu Gly Gly Gly Trp Gln Val Ser Leu Ile Ala Phe
                   170                 175                 180
   att acc gcg ttc acg atc att gcc acg acg tca ttt ttg gga aag aag    870
   Ile Thr Ala Phe Thr Ile Ile Ala Thr Thr Ser Phe Leu Gly Lys Lys
               185                 190                 195
   ggt ttg cct act ttc ttc caa aat gtt gtt ggt ggt ttt att gcc acg    918
   Gly Leu Pro Thr Phe Phe Gln Asn Val Val Gly Gly Phe Ile Ala Thr
           200                 205                 210
   ctg cct gca tcg att gct tat tct ttg gcg ttg caa ttt ggt ctt gag    966
   Leu Pro Ala Ser Ile Ala Tyr Ser Leu Ala Leu Gln Phe Gly Leu Glu
       215                 220                 225
   atc aaa ccg agc cag atc atc gca tct gga att gtt gtg ctg ttg gca   1014
   Ile Lys Pro Ser Gln Ile Ile Ala Ser Gly Ile Val Val Leu Leu Ala
   230                 235                 240                 245
   ggt ttg aca ctc gtg caa tct ctg cag gac ggc atc acg ggc gct ccg    1062
   Gly Leu Thr Leu Val Gln Ser Leu Gln Asp Gly Ile Thr Gly Ala Pro
                   250                 255                 260
   gtg aca gca agt gca cga ttt ttc gaa aca ctc ctg ttt acc ggc ggc    1110
   Val Thr Ala Ser Ala Arg Phe Phe Glu Thr Leu Leu Phe Thr Gly Gly
               265                 270                 275
   att gtt gct ggc gtg ggt ttg ggc att cag ctt tct gaa atc ttg cat    1158
   Ile Val Ala Gly Val Gly Leu Gly Ile Gln Leu Ser Glu Ile Leu His
           280                 285                 290
   gtc atg ttg cct gcc atg gag tcc gct gca gca cct aat tat tcg tct    1206
   Val Met Leu Pro Ala Met Glu Ser Ala Ala Ala Pro Asn Tyr Ser Ser
       295                 300                 305
   aca ttc gcc cgc att atc gct ggt ggc gtc acc gca gcg gcc ttc gca    1254
   Thr Phe Ala Arg Ile Ile Ala Gly Gly Val Thr Ala Ala Ala Phe Ala
   310                 315                 320                 325
   gtg ggt tgt tac gcg gag tgg tcc tcg gtg att att gcg ggg ctt act    1302
   Val Gly Cys Tyr Ala Glu Trp Ser Ser Val Ile Ile Ala Gly Leu Thr
                   330                 335                 340
   gcg ctg atg ggt tct gcg ttt tat tac ctc ttc gtt gtt tat tta ggc    1350
   Ala Leu Met Gly Ser Ala Phe Tyr Tyr Leu Phe Val Val Tyr Leu Gly
               345                 350                 355
   ccc gtc tct gcc gct gcg att gct gca aca gca gtt ggt ttc act ggt    1398
   Pro Val Ser Ala Ala Ala Ile Ala Ala Thr Ala Val Gly Phe Thr Gly
           360                 365                 370
   ggt ttg ctt gcc cgt cga ttc ttg att cca ccg ttg att gtg gcg att    1446
   Gly Leu Leu Ala Arg Arg Phe Leu Ile Pro Pro Leu Ile Val Ala Ile
       375                 380                 385
   gcc ggc atc aca cca atg ctt cca ggt cta gca att tac cgc gga atg    1494
   Ala Gly Ile Thr Pro Met Leu Pro Gly Leu Ala Ile Tyr Arg Gly Met
   390                 395                 400                 405
   tac gcc acc ctg aat gat caa aca ctc atg ggt ttc acc aac att gcg    1542
   Tyr Ala Thr Leu Asn Asp Gln Thr Leu Met Gly Phe Thr Asn Ile Ala
                   410                 415                 420
   gtt gct tta gcc act gct tca tca ctt gcc gct ggc gtg gtt ttg ggt    1590
   Val Ala Leu Ala Thr Ala Ser Ser Leu Ala Ala Gly Val Val Leu Gly
               425                 430                 435
   gag tgg att gcc cgc agg cta cgt cgt cca cca cgc ttc aac cca tac    1638
   Glu Trp Ile Ala Arg Arg Leu Arg Arg Pro Pro Arg Phe Asn Pro Tyr
           440                 445                 450
   cgt gca ttt acc aag gcg aat gag ttc tcc ttc cag gag gaa gct gag    1686
   Arg Ala Phe Thr Lys Ala Asn Glu Phe Ser Phe Gln Glu Glu Ala Glu
       455                 460                 465
   cag aat cag cgc cgg cag aga aaa cgt cca aag act aat cag aga ttc    1734
   Gln Asn Gln Arg Arg Gln Arg Lys Arg Pro Lys Thr Asn Gln Arg Phe
   470                 475                 480                 485
   ggt aat aaa agg taaaaatcaa cctgcttagg cgtctttcgc ttaaatagcg        1786
   Gly Asn Lys Arg
   tagaatatcg ggtcgatcgc ttttaaacac tcaggaggat ccttgccggc caaaatcacg 1846
   gacactcgtc ccaccccaga atcccttcac gctgttgaag aggaaaccgc agccggggta 1906
   ccg 1909
<210> 4
   <211> 489
   <212> PRT
   <213> Corynebacterium glutamicum ATCC13032
<400> 4
   Met Leu Ser Phe Ala Thr Leu Arg Gly Arg Ile Ser Thr Val Asp Ala
   1               5                   10                  15
   Ala Lys Ala Ala Pro Pro Pro Ser Pro Leu Ala Pro Ile Asp Leu Thr
               20                  25                  30
   Asp His Ser Gln Val Ala Gly Val Met Asn Leu Ala Ala Arg Ile Gly
           35                  40                  45
   Asp Ile Leu Leu Ser Ser Gly Thr Ser Asn Ser Asp Thr Lys Val Gln
       50                  55                  60
   Val Arg Ala Val Thr Ser Ala Tyr Gly Leu Tyr Tyr Thr His Val Asp
   65                  70                  75                  80
   Ile Thr Leu Asn Thr Ile Thr Ile Phe Thr Asn Ile Gly Val Glu Arg
                   85                  90                  95
   Lys Met Pro Val Asn Val Phe His Val Val Gly Lys Leu Asp Thr Asn
               100                 105                 110
   Phe Ser Lys Leu Ser Glu Val Asp Arg Leu Ile Arg Ser Ile Gln Ala
           115                 120                 125
   Gly Ala Thr Pro Pro Glu Val Ala Glu Lys Ile Leu Asp Glu Leu Glu
       130                 135                 140
   Gln Ser Pro Ala Ser Tyr Gly Phe Pro Val Ala Leu Leu Gly Trp Ala
   145                 150                 155                 160
   Met Met Gly Gly Ala Val Ala Val Leu Leu Gly Gly Gly Trp Gln Val
                   165                 170                 175
   Ser Leu Ile Ala Phe Ile Thr Ala Phe Thr Ile Ile Ala Thr Thr Ser
               180                 185                 190
   Phe Leu Gly Lys Lys Gly Leu Pro Thr Phe Phe Gln Asn Val Val Gly
           195                 200                 205
   Gly Phe Ile Ala Thr Leu Pro Ala Ser Ile Ala Tyr Ser Leu Ala Leu
       210                 215                 220
   Gln Phe Gly Leu Glu Ile Lys Pro Ser Gln Ile Ile Ala Ser Gly Ile
   225                 230                 235                 240
   Val Val Leu Leu Ala Gly Leu Thr Leu Val Gln Ser Leu Gln Asp Gly
                   245                 250                 255
   Ile Thr Gly Ala Pro Val Thr Ala Ser Ala Arg Phe Phe Glu Thr Leu
               260                 265                 270
   Leu Phe Thr Gly Gly Ile Val Ala Gly Val Gly Leu Gly Ile Gln Leu
           275                 280                 285
   Ser Glu Ile Leu His Val Met Leu Pro Ala Met Glu Ser Ala Ala Ala
   290                 295                 300
   Pro Asn Tyr Ser Ser Thr Phe Ala Arg Ile Ile Ala Gly Gly Val Thr
   305                 310                 315                 320
   Ala Ala Ala Phe Ala Val Gly Cys Tyr Ala Glu Trp Ser Ser Val Ile
                   325                 330                 335
   Ile Ala Gly Leu Thr Ala Leu Met Gly Ser Ala Phe Tyr Tyr Leu Phe
               340                 345                 350
   Val Val Tyr Leu Gly Pro Val Ser Ala Ala Ala Ile Ala Ala Thr Ala
           355                 360                 365
   Val Gly Phe Thr Gly Gly Leu Leu Ala Arg Arg Phe Leu Ile Pro Pro
       370                 375                 380
   Leu Ile Val Ala Ile Ala Gly Ile Thr Pro Met Leu Pro Gly Leu Ala
   385                 390                 395                 400
   Ile Tyr Arg Gly Met Tyr Ala Thr Leu Asn Asp Gln Thr Leu Met Gly
                   405                 410                 415
   Phe Thr Asn Ile Ala Val Ala Leu Ala Thr Ala Ser Ser Leu Ala Ala
               420                 425                 430
   Gly Val Val Leu Gly Glu Trp Ile Ala Arg Arg Leu Arg Arg Pro Pro
           435                 440                 445
   Arg Phe Asn Pro Tyr Arg Ala Phe Thr Lys Ala Asn Glu Phe Ser Phe
       450                 455                 460
   Gln Glu Glu Ala Glu Gln Asn Gln Arg Arg Gln Arg Lys Arg Pro Lys
   465                 470                 475                 480
   Thr Asn Gln Arg Phe Gly Asn Lys Arg
                   485